Europäisches Patentamt

European Patent Office (11) Publication number: **0 173 480**
Office européen des brevets **A2**

(19)

(12) **EUROPEAN PATENT APPLICATION**

(21) Application number: 85305559.8

(51) Int. Cl.⁴: **A 61 K 31/70**

(22) Date of filing: 05.08.85

(30) Priority: 30.08.84 US 646176

(43) Date of publication of application: 05.03.86
Bulletin 86/10

(84) Designated Contracting States: BE CH DE FR GB IT LI
NL SE

(71) Applicant: THE UPJOHN COMPANY, 301 Henrietta
Street, Kalamazoo, Michigan 49001 (US)
Applicant: ARIZONA STATE UNIVERSITY, Tempe
Arizona (US)

(72) Inventor: Wierenga, Wendell, c/o The Upjohn
Company 301 Henrietta Street, Kalamazoo
Michigan 49001 (US)
Inventor: Pettit, George Robert, 6232 Bret Hills Drive,
Paradise Valley Arizona 85251 (US)
Inventor: Renis, Harold Edward, c/o The Upjohn
Company 301 Henrietta Street, Kalamazoo
Michigan 49001 (US)

(74) Representative: Perry, Robert Edward et al, GILL
JENNINGS & EVERY 53-64 Chancery Lane, London
WC2A 1HN (GB)

(54) **The antiviral use of phyllanthostatins.**

(57) The use of a compound selected from phyllanthostatin I,
phyllanthoside and phyllantocin, for the manufacture of a
medicament for treating or preventing a virus in a mammal
suffering from or susceptible to the virus.

EP 0 173 480 A2

ACTORUM AG

## THE ANTIVIRAL USE OF PHYLLANTHOSTATINS

The present invention relates to a new use of phyllanthostatins.

The phyllanthostatins and phyllanthosides are disclosed in EP-A-0087861 (and US-A-4388457) as anti-neoplastic agents. They are isolated from the Central American tree Phyllanthus acuminatus Vahl. The structures of these compounds are given on pages 24 and 25 of EP-A-0087861.

The viruses are the smallest of the parasites. They consist of intracellular molecular particles, in some instances crystallisable, with a central core of nucleic acid and an outer core of protein. They are wholly dependent upon cells (bacterial, plant or animal) for reproduction. The nucleic acid core (either RNA or DNA) represents the basic infectious material, being able in many cases to penetrate susceptible cells and to initiate infection alone. Several hundred different viruses may infect humans. Many have been recognised only recently, and their clinical effects or relationships are not fully delineated. Many other viruses usually produce non-evident infections and only occasionally overt disease; nevertheless, because of their wide (sometimes universal) prevalence and their numerous distinct serotypes, they create important medical and public health problems. See The Merck Manual, 13th Edition, Section 1, Chapter 2, pages 11-18 (1977). While there are many vaccines, available for general use, effectively prophylactic against viruses such as influenza, common measles, common mumps, poliomyelitis, rabies, rubella, smallpox and yellow fever, there are few agents which effectively treat viral infections in mammals, especially humans. See The Merck Manual, supra.

The present invention is based on the discovery of the utility of a method of treating or preventing a viral disease in a mammal suffering from or susceptible to said disease which comprises systemically administering a compound selected from the group consisting of phyllanthostatin I, phyllanthoside, or phyllanthocin to said mammal in an amount effective to treat or prevent said viral disease.

The isolation, characterization, and formulation of the phyllanthostatin, phyllanthoside, and phyllanthocin are described in U.S. Patent 4,388,457, which is expressly incorporated herein by reference. The structures are shown in Chart A.

The administration of phyllanthostatin, phyllanthoside, and phyllanthocin are useful prophylactically and therapeutically for treating viral diseases. By "viral disease" or "viral infection" is meant any disease or condition caused by intracellular molecular particles with a nucleic acid core (RNA or DNA), such as hepatitis virus, rubella, rubeola, influenza, encephalitis viruses (i.e., arboviruses such as western or eastern equine encephalitis virus, Semliki Forest virus), herpes viruses (types 1 or 2 herpes simplex virus, cytomegalovirus, varicella-zoster and infectious bovine rhinotracheitis virus), rabies, enteroviruses (picornaviruses, echoviruses, coxsackie viruses), para-influenza viruses, respiratory syncytial virus, sendai virus, poliomyelitis viruses, yellow fever, Epstein-Barr virus (infectious mononucleosis), small pox, Dengue virus, common cold virus (rhinoviruses, coronaviruses, etc.), adenoviruses, polyomaviruses, papovaviruses, RNH-tumor viruses (e.g., feline leukemia virus, avian leukosis virus, avian sarcoma viruses), B virus, aleutians disease of mink, arena viruses, blue tongue virus of sheep, bovine viral diarrhea-mucosal disease virus, canine distemper virus, canine hepatitis virus, canine herpesvirus, equine abortion virus, infectious equine anemia virus, fowl pox virus, hog cholera virus, Marek's disease, mink enteritis virus, Newcastle disease virus, porcine enterovirus, pseudorabies virus, foot and mouth disease virus, reoviruses, and all other viruses or diseases of viral origin (for example, slowly progressing diseases that may be of viral origin such as multiple sclerosis).

The present invention includes the treatment of each of various mammalian species, including humans. Humans are particularly preferred in this invention. With respect to non-humans, the present invention is particularly and especially concerned with treating domesticated animals, for example, cattle, dogs, cats, and swine. By treatment is meant any alleviation of the symptoms caused by viral diseases or infections. By prevention is meant partial or total avoidance of the symptoms of a viral disease, depending on the severity of the disease.

Any convenient route of administration is employed. Thus, oral formulation and oral administration is, for example, the preferred route for use in humans although parenteral (e.g., intravenous, intraperitoneal, intramuscular) administration is also employed.

The dosage administered will be dependent upon the identity of the viral disease or infection, the type of host involved, its age, health, weight, kind of concurrent treatment, if any, frequency of treatment and therapeutic ratio.

Illustratively, dosage levels of the administered active ingredients can be: intravenous 0.01 to about 20 mg/kg; intraperitoneal, 0.01 to about 100 mg/kg; subcutaneous, 0.01 to about 100 mg/kg; intramuscular, 0.01 to about 100 mg/kg; orally, 0.01 to about 200 mg/kg, and preferably about 1 to 100 mg/kg; intranasal instillation, 0.01 to about 20 mg/kg; and aerosol, 0.01 to about 20 mg/kg of animal (body) weight.

Expressed in terms of concentration, an active ingredient can be present in the compositions of the present invention for localized use about the cutis, intranasally, pharyngolaryngeally, bronchially, broncholially, intravaginally, rectally, or ocularly in a concentration of from about 0.01 to about 20% w/w of the composition; and for parenteral use in a concentration of from about 0.05 to about 50% w/v of the composition and preferably from about 5 to about 20% w/v.

The compositions of the present invention are preferably presented for administration to humans and animals in unit dosage forms, such as tablets, capsules, pills, powders, granules, suppositories, sterile parenteral solutions or suspensions, sterile non-parenteral solutions

0173480

171

or suspensions, and oral solutions or suspensions and the like, containing suitable quantities of an active ingredient.

For oral administration either solid or fluid unit dosage forms can be prepared.

Powders are prepared quite simply by comminuting the active ingredient to a suitably fine size and mixing with a similarly comminuted diluent. The diluent can be an edible carbohydrate material such as lactose or starch. Advantageously, a sweetening agent or sugar is present as well as a flavoring oil.

Capsules are produced by preparing a powder mixture as hereinbefore described and filling into formed gelatin sheaths. Advantageously, as an adjuvant to the filling operation, a lubricant such as a talc, magnesium stearate, calcium stearate and the like is added to the powder mixture before the filling operation.

Soft gelatin capsules are prepared by machine encapsulation of a slurry of active ingredients with an acceptable vegetable oil, light liquid petrolatum or other inert oil or triglyceride.

Tablets are made by preparing a powder mixture, granulating or slugging, adding a lubricant and pressing into tablets. The powder mixture is prepared by mixing an active ingredient, suitably comminuted, with a diluent or base such as starch, lactose, kaolin, dicalcium phosphate and the like. The powder mixture can be granulated by wetting with a binder such as corn syrup, gelatin solution, methyl-cellulose solution or acacia mucilage and forcing through a screen. As an alternative to granulating, the powder mixture can be slugged, i.e., run through the tablet machine and the resulting imperfectly formed tablets broken into pieces (slugs). The slugs can be lubricated to prevent sticking to the tablet-forming dies by means of the addition of stearic acid, a stearic salt, talc or mineral oil. The lubricated mixture is then compressed into tablets.

Advantageously the tablet can be provided with a protective coating consisting of a sealing coat or enteric coat of shellac, a coating of sugar and methylcellulose and polish coating of carnauba wax.

Fluid unit dosage forms for oral administration such as syrups, elixirs and suspensions can be prepared wherein each teaspoonful of composition contains a predetermined amount of active ingredient for administration. The water-soluble forms can be dissolved in an aqueous vehicle together with sugar, flavoring agents and preservatives to form a syrup. An elixir is prepared by using a hydroalcoholic vehicle with suitable sweeteners together with a flavoring agent. Suspensions can be prepared of the insoluble forms with a suitable vehicle with the aid of a suspending agent such as acacia, tragacanth, methylcellulose and the like.

For parenteral administration, fluid unit dosage forms are prepared utilizing an active ingredient and a sterile vehicle, water being preferred. The active ingredient, depending on the form and concentration used, can be either suspended or dissolved in the vehicle. In preparing solutions the water-soluble active ingredient can be dissolved in water for injection and filter sterilized before filling into a suitable vial or ampule and sealing. Advantageously, adjuvants such as a local anesthetic, preservative and buffering agents can be dissolved in the vehicle. Parenteral suspensions are prepared in substantially the same manner except that an active ingredient is suspended in the vehicle instead of being dissolved and sterilization cannot be accomplished by filtration. The active ingredient can be sterilized by exposure to ethylene oxide before suspending in the sterile vehicle. Advantageously, a surfactant or wetting agent is included in the composition to facilitate uniform distribution of the active ingredient.

In addition to oral and parenteral administration, the rectal and vaginal routes can be utilized. An active ingredient can be administered by means of a suppository. A vehicle which has a melting point at about body temperature or one that is readily soluble can be utilized. For example, cocoa butter and various polyethylene glycols (Carbowaxes) can serve as the vehicle.

For intranasal instillation, fluid unit dosage forms are prepared utilizing an active ingredient and a suitable pharmaceutical vehicle, water being preferred, or by dry powder for insufflation.

The active ingredients can also be admixed in animal feed. The active ingredients can conveniently be prepared in the form of a food premix. The food premix can comprise an active ingredient in admixture with an edible pharmaceutical diluent such as starch, oatmeal, flour, calcium carbonate, talc, dried fish meal and the like non-toxic, orally acceptable pharmaceutical diluents. The prepared premix is then conveniently added to the regular feed.

For use as aerosols the active ingredients can be packaged in a pressurized aerosol container together with a gaseous or liquefied propellant, for example, dichlorodifluoromethane, carbon dioxide, nitrogen, propane, and the like, with the usual adjuvants such as co-solvents and wetting agents, as may be necessary or desirable.

The term "unit dosage form" as used in the specification and claims refers to physically discrete units suitable as unitary dosages for human and animal subjects, each unit containing a predetermined quantity of active material calculated to produce the desired therapeutic effect in association with the required pharmaceutical diluent, carrier or vehicle. The specifications for the novel unit dosage forms of this invention are dictated by and are directly dependent on (a) the unique characteristics of the active material and the particular therapeutic effect to be achieved, and (b) the limitation inherent in the art of compounding such an active material for therapeutic use in humans, as disclosed in this specification, these being features of the present invention. Examples of suitable unit dosage forms in accord with this invention are tablets, capsules, troches, suppositories, powder packets, wafers, cachets, teaspoonfuls, tablespoonfuls, dropperfuls, ampuls, vials, segregated multiples of any of the foregoing, and other forms as herein described.

The active ingredients to be employed as anti-viral agents can be easily prepared in unit dosage form with the employment of pharmaceutical materials which themselves are available in the art and can be prepared by established procedures.

DESCRIPTION OF THE PREFERRED EMBODIMENTS

The antiviral activity of the instantly claimed phyllanthostatin I, phyllanthoside, and phyllanthocin is seen by the following examples:

Example 1    In vitro Studies on Antiviral and Cytotoxic Properties

These tests were performed by the methods described in the following published reference: H.E. Renis, C.A. Hollowell, and G.E. Underwood, J. Med. Chem., Vol. 10, pp. 777-782 (1967)). Both DNA and RNA-containing viruses were used. The results are presented below as the zone of cytotoxicity (cell death)/zone of viral plaque inhibition for 50 μg

of test compound. The in vitro antiviral activity and cytotoxicity of the phyllanthostatins, including the aglycone (phyllanthocin) have been evaluated. Of the compounds evaluated, several were effective in the cell culture assay system. Phyllanthoside, phyllanthocin, and phyllanthostatin I were inhibitory for the replication of herpes simplex viruses types 1 and 2. Phyllanthoside and phyllanthostatin I were equipotent, and approximately five times more active than phyllanthocin. These compounds were less inhibitory for RNA virus replication. These same compounds were cytotoxic for growing Vero cells during 72 hours of exposure, but greater exposure times suggested that cell growth resumed, perhaps by drug inactivation. Drug cytotoxicity was determined by measuring the OD260nm of cell lysates. The therapeutic indices of these compounds were found to be about 0.5-0.8 for HSV-1 and HSV-2.

Methods

1.    Cell culture antiviral activity: Monolayers of appropriate cells and 100 mm plastic culture dishes are infected with suitably diluted virus. After the virus has sttached to the cells (60 min, 37°) an agar-199-fetal bovine serum medium is added (15 ml) to the infected cultures and allowed to solidify. Walls (1/4 inch diameter) are cut from the agar, into which approximately 50 µl of drug containing solution or suspension is placed. The dishes are incubated at 37° for an interval which allows virus plaque formation. Antiviral activity is determined after straining the monolayers by zones free of plaque formation. Cytotoxicity is evident by clear zones representing cell lysis. The results are expressed as zones of cytotoxicity/zones of antiviral activity. The zones sizes are given as 1-4, where 1 = zone size to 10-20 mm, 2 = 20-30 mm, etc.

2.    Drug preparation: Stocks of drug were prepared at 20 mg per ml and dimethyl sulfoxide (DMSO), and further diluted in DMSO so that 50 µl, when diluted in 20 ml BMD-3% fbs, gave the drug concentrations indicated.

3.    Inhibition of virus replication: Vero cells were used for the replication of herpes simplex virus type 1 (HSV-1, McIntyre), herpes simplex virus type 2 (HSV-2, MS) and VSV (vesicular stomatitis virus). ML cells were used for coxsackie A21 studies. Monolayer cultures (35 mm plastic culture dishes) were infected with the appropriate virus. The infected cultures, after washing with Hank's solution to remove non-absorbed virus, were refed with 2 ml BME-3% fbs plus antibiotics containing drugs at the indicated concentration. The cultures were incubated for the indicated times, and harvested (-70°). Virus yields were determined by the plaque method on primary rabbit kidney monolayer (HSV-1 and HSV-2), L929 monolayers (VSV) or ML monolayers (coxsackie A21).

4. Cytotoxicity of drugs: Phyllanthostatin cytotoxicity was determined by following the change in absorbance at 260 nm in lysed cell monolayers. Vero cells ($5 \times 10^4$ cells per ml in EMEM plus 10% fbs plus antibiotics) were seeded (2 ml) in 35 mm plastic culture dishes, and incubated at 37° for 4 hours to allow for cell attachment. The drugs (20 mg per ml in DMSO) to be studied were diluted in DMSO and finally in EMEM plus 10% fbs (150 µl of DMSO solution in 30 ml medium) so that the final drug concentrations indicated was achieved. At appropriate intervals, 6 controls (with DMSO) or 3 drugs containing dishes were washed 3 times with ice cold phosphate buffered saline. Cell lysis was accomplished by adding 1 ml of a solution containing 0.01 M echoline ethylene diamine tetracetic acid (EDTA), 1% sodium dodecyl sulfate in 0.01 M tris buffer (pH 7.7). After incubation at 25° for 1 hour, the cell lysate was withdrawn and stored at 4° with 1 ml of the water used to wash the culture dish. The absorbance at 260 nm was then determined.

5. Calculation of T values (therapeutic index): For a given compound, the T value is the $ID_{50}$ (drug dose which reduces the OD at 260 nm in the cell lysate by 50%) divided by the $ED_{90}$ (that does which reduces the virus yield by 90%). The larger the therapeutic index (T) the greater the selectivity of the drug for virus inhibition.

Results

A. Cell culture antiviral activity: The results of the cell culture testing of the phyllanthostatin compounds are shown in Table 1.

B. Inhibition of virus replication: At drug concentrations of 0.5 µg per ml, none of the drugs reduced yields of coxsackie A21 and only slight reductions and titers were observed with VSV and HSV-1. At this drug concentration, HSV-2 appeared to be the most susceptible virus to inhibition; particularly with phyllanthoside and phylanthostatin I. At higher drug concentrations (5 and 50 μg per ml) further reductions in all virus yields were observed with phyllanthoside and phyllanthostatin I. Phyllanthocin was non-inhibitory for VSV and coxsackie A21 at concentrations below 5 μg per ml and only modestly inhibitory for HSV-1, while HSV-2 yields were reduced by greater than 1 log at this concentration. Phyllanthocin (50 μg per ml) reduced yields of all viruses. These data suggest that phyllanthocin may be selectively inhibitory for HSV-1 and HSV-2, and that phyllanthoside and phyllanthostatin I are equipotent for all viruses included in this study.

Another study using different drug concentrations and determining the titers of HSV-1, HSV-2, and VSV yields from infected Vero cells, confirms that phyllanthoside and phyllanthostatin I are nearly equipotent and more inhibitory than phyllanthocin and that all drugs appear to be more inhibitory for HSV-2 than the other viruses. VSV appears to be the least sensitive and HSV-1 is intermediate in sensitivity.

To determine the efficacy of the phyllanthostatins for picorna virus replication in Vero cells, so as to relate it to the herpes virus inhibition, coxsackie B3 virus (Nancy Strain) was utilized. The virus yields from ML and Vero cells infected with coxsackie A21 and coxasckie B3, respectively, and treated with diffeent concentrations of the phyllanthostatins, revealed that phyllanthoside and phyllanthostain I and were inhibitory for both viruses at 5 μg per ml. At concentrations below 5 μg per ml (i.e., 0.5 or 1 μg per ml) reduced titers of coxsackie A21 resulted from ML cells, while at the same drug concentrations coxsackie B3 yields were unaffected. Phyllanthocin at 5 μg per ml was inhibitory for coxsackie B3 replication in Vero cells, but did not reduce the yields of coxsackie A21 virus in ML cells.

C. Cytotoxicity determination: At the highest drug level, essentially no change in OD260 occurred during the intervals studied. At concen-trations og 1 μg per ml with phyllanthoside and phyllanthostatin I and 5 μg per ml with phyllanthocin cell growth was suppressed to a greater extent during the first 72 hours; then during the next day (96 hours), the OD260 increased dramatically compared to the 48 hour control cells. Phyllantoside and phyllanthostatin I at 0.5 μg per ml decreased the OD260 during 72 hours, but, this phenomenon occurred again after 72 hours with a greatly increased OD260 during the next day of incubation. Lesser concentrations of drug were only marginal cytotoxic by these criteria.

D.   Therapeutic index calculations:   The therapeutic index for phyllanthoside, phyllanthocin, and phyllanthostatin I were determined based upon the antiviral activity and the cytotoxicity. The antiviral activity is the drug concentration required to reduce the virus titers by 90% ($ED_{90}$) from cultures infected with a multiplicity of greater than 1, i.e., each cell is infected. The cytotoxicity ($ID_{50}$) is the drug concentration which reduces the OD260 of lysed cells by 50%. Because of the change in OD260 between 72 and 96 hours, both values were used in calculating the therapeutic index (Table 2). The $ID_{50}$'s at 72 hours, were 1.195, 5.91 and 1.21 mg per ml of phyllanthoside, phyllanthocin, and phyllanthostatin I, respectively. At 96 hours, the $ID_{50}$'s had increased to 3.57, 7.09 and 3.64 μg per ml, respectively. The $ED_{90}$'s for HSV-1 were 1.87, 7.14 and 2.37 mg for phyllanthoside, phyllanthocin, and phyllanthostatin I; while those for HSV-2 were 1.50, 7.99 and 1.64 μg per ml for phyllanthoside, phyllanthocin, and phyllanthostatin I, respectively. These data reveal that these compounds were slightly more inhibitory for HSV-2 than HSV-1 and further that phyllanthocin was about 2-5 times less cytotoxic than phyllanthoside or phyllanthostatin I and 2-3 times less inhibitory for HSV-1 and HSV-2.

E.   Drug stability studies: The changes in OD260 in lysed cells treated with different drug concentrations could come about if the cells became resistant to drug during the interval of incubation or if the drug was unstable at 37° in the cell culture medium. To test the latter, freshly prepared drug stocks in DMSO were appropriately diluted in EMEM-10% fbs with antibiotics. These preparations were divided and either incubated at 37° or stored at -40° for 96 hours. These media were then compared with freshly prepared drug for the affects on cell growth. The results of the cytotoxicity studies comparing freshly prepared drug and medium, drug containing medium stored at -40°, and drug containing medium incubated at 37° show that the cytotoxicity is unchanged if drug containing medium is stored at -40° compared to freshly prepared drug no matter which drug is used. In contrast, incubation of the drug at 37° for 96 hours completely destroyed the cytotoxicity of all 3 drugs.

The drug stability studies were extended to include comparative antiviral (HSV-1 and HSV-2) activity of drug and medium stored at -40° for 96 hours with that of drug and medium incubated at 37° for 96 hours. Drugs prepared in BME-3% fbs and incubated at -40° for 96 hours were inhibitory for HSV-1 to nearly the same extent as previously observed. However, after 96 hours at 37°, none of the drugs reduced the virus yields at the same concentrations studied. The fact that the same virus yields were obtained with control BME-3% fbs (i.e., BME-3% fbs with the same concentration of DMSO as in the drug containing medium) indicates that under these conditions the medium did not become incapable of supporting HSV-1 replication. Similar results were observed with the same drug/medium mixtures regarding HSV-2 replication. Drugs and medium incubated at 37° for 96 hours showed greatly reduced activity regarding HSV-2 yields. With HSV-2, higher concentrations (10 µg/ml) of drug and medium incubated at 37° all brought about 0.5-1.0 log reductions in virus titers, but lower drug concentrations were noninhibitory. Phyllanthoside and phyllanthostatin I in medium stored at -40° were nearly equal in potency, and more inhibitory at the same drug dose than was phyllanthocin.

Example 2      In Vivo Studies of Effect of Genital HSV-2 (Herpes Simplex Virus Type II) Infection of Female Mice When Drugs are Given Intravaginally After Virus Inoculation These same compounds were evaluated in female mice with genital HSV-2 infection. Phyllanthoside was the most effective of the compounds, although phyllanthostatin I protected mice from lethal infection when the virus inoculum was reduced. Phyllanthocin was devoid of antiviral activity in this model. The drugs were delivered intravaginally to mice shortly (one hour) after intravaginal inoculation of HSV-2, and treatments were continued 3 times daily.

Additional studies showed that the drug concentrations which resulted in antiviral activity did not affect weight gain of the mice. Pathogenesis studies revealed that the protection of mice from virus caused death was related to the inhibition of virus replication in the vagina. Phyllanthoside treatment of mice resulted in reduced isolation rates and titers of HSV-2 in the vaginal secretions. Such reduction in the vaginal HSV-2 titers in mice treated with phyllanthostatin I was only transient, although mean virus titers were somewhat less than that observed in those mice treated intravaginally with the vehicle.

Methods

Mice:    Female mice were housed in hanging stainless steel cages and provided water and feed (Purina Lab Chow) ad libitum. At the initiation of the experiment the mice weighed 16-18 grams.

Virus:    Herpes Simplex virus type 2 (HSV-2, strain 35D) was passaged in and assayed (plaque method) on primary rabbit kidney monolayers. The titer of the stock virus used in these studies was 1.6 x $10^6$ PFU per 0.2 ml. Where indicated, virus dilutions were made in Eagles Basal Medium supplemented with 3% fetal bovine serum and antibiotics (BME-3% fbs).

Drugs:    Drugs were sobulized in DMSO at 10 or 20 mg/ml. These stocks were further diluted in 0.9% sodium chloride (NaCl) to the concentrations indicated.

Experimental Protocol:    One hour after swabbing the vaginas, 0.1ml, HSV-2 was inoculated intravaginally. Intravaginal drug or placebo treatment (0.1 ml) was initiated 1 hour following virus inoculation. Two additional treatments were given on the day of infection; one 3 hours and a second 7 hours following virus inoculation. On subsequent days, intravaginal drug treatments were given at 8 a.m., noon and 4 p.m.

Where indicated, non-infected mice (5 per group) were treated identically (3 times daily x 5) in order to determine drug toxicity. The groups of mice were weighed at the indicated times.

The pathogenesis of the infection was followed by determining the virus titers in vaginal secretions. Vaginal swabs were collected from each of the mice on days 1, 2, 3, 4 and 7 following infection. Each swab was placed in 2 ml BME-3% fbs and stored at -70° C until assayed for a virus by the plaque method.

Illnesses and deaths were recorded daily. The percent survivors (%S) and mean day of death (MDD) calculated (3). The probability that differences in % S or MDD was due to chance was determined by the Fisher Exact Test and Chi-Square Analysis, respectively.

Results: The studies summarized in Table 3 show the survivors in groups of female mice inoculated with HSV-2 and treated intravaginally with the phyllanthostatins or saline. In this study, zinc acetate (0.1M) was included as a positive control. Mice were inoculated with either $8.0 \times 10^4$ or $8.,0 \times 10^3$ PFU in 0.1 ml. These inocula were selected using the rationale that "true" antiviral activity, if present in animals inoculated at the greater inoculum rate would be magnified then in those animals where the inoculum was reduced. Eleven and 12 of 14 mice in the saline treated groups died when inoculated with $8.0 \times 10^4$ and $8.0 \times 10^3$ PFU respectively. However, the mean day of death was increased from 7.0 to 9.0 days with the reduction in inoculum rate. Seven (50%) and three (21.4%) deaths were observed in the groups of mice treated with zinc acetate in the groups inoculated with $8.0 \times 10^4$ and $8.0 \times 10^3$ PFU respectively. It should be noted that in the latter two of the three deaths occurred on day 4 and were probably not virus related. The reason for these early deaths in the zinc acetate treated group is unknown but have been observed in other studies.

At the drug doses used, phyllanthoside was the most active of those studied. Mice treated intravaginally with phyllanthoside and inoculated with $8.0 \times 10^4$ or $8.0 \times 10^3$ PFU in 0.1 ml were differentially protected in that 9 and 13 (of 14) mice survived respectively. In contrast, phyllanthostatin I, a compound equipotent with phyllanthoside in cell culture studies allowed for survival of 4 and 10 mice respectively in the groups inoculated with a $8.0 \times 10^4$ or $8.0 \times 10^3$ PFU respectively. Phyllanthocin did not protect mice from lethal infection when the inoculum was $8.0 \times 10^4$ PFU. When the inoculum was reduced to $8.0 \times 10^3$ PFU, 5 mice survived, four mice survived in the group treated with phyllanthocin. The statistical analysis of these data is shown in Table 3.

The effect of phyllanthoside and phyllanthostatin I was further studied in order to determine whether the differences in activity (Table 1 and Figure 1) could be better understood. Mice were inoculated intravaginally with HSV-2 ($8.0 \times 10^4$ PFU in 0.1 ml) and treated intravaginally with drugs. All mice (10 of 10) died in the group treated with 0.9% NaCl and 8 (of 9) died in the DMSO (10%) in 0.9% NaCl. Zinc acetate, the positive control, protected 80% of the mice from lethal infection, i.e., 2 of 10 mice died. Again, one of the deaths occurred early after infection (on day 3) and was probably not associated with viral disease. Phyllanthoside at a concentration of 1 mg/ml protected 7 of 10 mice from death, however, at a lower dose (i.e., 0.5 mg/ml), only 1 mouse survived. When mice were treated with phyllanthostatin I at 1 mg/ml death occurred in 80% of the mice, and when the dose was reduced to 0.5 mg/ml, all of the animals succumed to infection. The analysis of the percent survivors and the mean day of death are shown in Table 4.

One day following HSV-2 inoculation, virus was recovered from the vaginal swabs of 8 to 10 mice treated with saline and 8 of 9 swabs from mice treated with DMSO (10%) in saline. The mean titers (log PFU/ml) of those swabs containing virus were 3.9 and 3.5 respectively at this time. All saline treated mice showed virus after this time and on days 2, 3 and 4, the mean virus titers were 4.4, 4.3, and 4.3 respectively. All animals in the DMSO-saline treated group shed virus on day 2 (mean virus titer 4.4). On days 3 and 4, 9 of 10 mice shed virus, and the mean titers were 4.3 at both of these times. One of 10 mice treated with zinc acetate shed virus on day 1, and continued through day 4. Thus, the protection of mice from lethality by zinc acetate treatment was further reflected by the reduced rate of virus isolation from the vaginal secretions.

Treatment with phyllanthoside (1 mg/ml) reduced the isolation rate of virus; 2, 3, 5, and 4 of 10 mice shed virus on days 1, 2, 3 and 4 respectively. The mean titers in this group was also about one log less in days 1, 2 and 3. One day 4, the mean titer was bout 0.6 logs less than that of the saline or DMSO-saline treated groups. It should be noted that mice were receiving treatment during this time interval, yet the number of mice shedding virus, and the mean titers increased. When the phyllanthoside dose was reduced to 0.5 mg/ml, virus was recovered from 5 to 10 mice on day 1, and 7 of 8 (of 10) mice thereafter. Again, there was a daily increase in the mean virus titer, although the mean titers were again about 1 log less than the placebo treated groups for the first 3 days, then the difference was about 0.6 logs.

Initially, treatment with phyllanthostatin I (1 mg/ml) reduced the number of mice from which virus could be isolated; on day 1 virus was isolated from 4 of 10 swabs, hoever 8 of 10 swabs contained virus after this time. The mean virus titers remained lower (about 0.5 logs) on days 2 through 4 compared to the placebo treated controls where on day 1 the mean titer was greater than 2 logs less than the placebo.

When the dose of phyllanthostatin I was reduced to 0.5 mg/ml, the number of mice shedding virus in the vaginal secretions was the same as that in the group treated with saline or DMSO saline. However, at this lower dose, the mean virus titers were about one log lower than the placebo treated group on day 1, and on days 2 and 3 the titers were about 0.7 logs less. On day 4, the mean titers of the group treated with phyllanthostatin I at 0.5 mg/ml was nearly the same as that in the controls. At this dose of phyllanthostatin I, the MDD was the same as the saline and DMSO saline treated group (Table 4).

Non-infected female mice were treated intravaginally with the same drugs, at the same concentrations and with a schedule (3 x daily x 5 days). Animal weights were determined at the time of initiation of treatment (day 0), and again on day 4 (after 12 treatments were given), and again after treatments had been concluded on days 7, 11 and 15. The weights of the groups of mice treated with placebo or the high drug concentrations are shown on Table 3, and the differences in weights in Figure 4. Weight changes in groups of mice treat4ed with saline, (1 mg/ml in DMSO saline) and zinc acetate were essentially the same. Weight gains in mice treated with phyllanthoside (1 mg/ml) were slightly less than that of the above compounds, but by day 15, the mice had the same gain as those treated with phyllanthostatin I, saline or zinc acetate. The poorest weight gain was observed in those mice treated with DMSO (saline 10%). No deaths were observed in any of the groups of mice.

Example 3        Cell Culture Testing

Phyllanthoside and Phyllanthostatin I were nearly identical in potency in reducing the yields of VSV, Coxsackie A-21, HSV-1 or its SU-2 virus in cell culture. Phyllanthocin, the aglycone methyl ester of phyllanthoside was moderate in activity; reductions in yields of

0173480

both HSV-1 and HSV-2 were observed at 5 µg/ml, while the titers of VSV and Coxsackie A-21 were not affected at this concentration. Higher concentrations of Phyllanthocin, were inhibitory for all viruses.

Methods: Vero cells were used for the replication of HSV-1, HSV-2 and VSV.ML cells were used with Coxsackie A-21 virus. Mono-layer cell cultures were infected with the appropriate virus. The infected cultures, after washing with Hanks to remove unabsorbed virus were refed with BME-3% fbs containing either 0, 0.5, 5.0 or 50.0 µg/ml drug. Stocks of drugs were prepared at 20 mg/ml in DMSO, and further diluted in DMSO so that 50 µl, when diluted in 20 ml BME-3% fbs, gave the drug concentrations given. The cultures were incubated at 37° overnight (for Coxsackie A-21 and VSV) or 24 hours for HSV-1 and HSV-2. Virus yields were determined by the plaque method on primary rabbit kidney monolayers (HSV-1 and HSV-2), L929 monolayers (VSV), or ML monolayers (Coxsackie A-21).

Results and Discussion: At 0.5 µg/ml, none of the drugs was inhibitory for Coxsackie A-21 virus, and only slight reductions in titers were observed with VSV and HSV-1. At this concentration, HSV-2 appeared to be the most susceptible virus to inhibition; particularly with Phyllanthoside and Phyllanthostatin I. At higher drug concentrations (5 and 50 Yg/ml) further reductions in all virus yields were observed with Phyllanthoside and Phyllanthostatin I. At 5 µg/ml, Phyllanthocin aglycone methyl ester of phyllanthoside was inhibitory for both HSV-1 and HSV-2, but not for VSV or Coxsackie A-21, while reduction in titers of all viruses were observed with 50 µg/ml. Phyllanthostatin 3 at 50 µg/ml caused slight reduction in yields of HSV-1 and HSV-2, but not in the yields of Coxsackie A-21 and VSV. These data, although preliminary, suggest that Phyllanthocin, aglycone methyl ester of phyllanthoside is a selective inhibitor of DNA (HSV-1 and HSV-2) virus replication.

4171

## TABLE 1

| Compound | PR-8[a] | Coe | VIRUS: HA-1 | E.R. | HSV-1 | HSV-2 | Vacc. |
|---|---|---|---|---|---|---|---|
| Phyllanthoside | 3/3 | 3/0 | 4/0 | 2/4 | 4/4 | 3/4 | 4/4 |
| Phyllanthocin | 2/0 | 0/0 | 0/0 | 0/0 | 0/3 | 0/2 | 0/4 |
| Phyllanthostatin I | 2/2 | 4/0 | 4/0 | 2/4 | 4/4 | 4/4 | 4/4 |

————

[a]PR-8 is influenza virus A, Coe is Coxsackie A-21, HA-1 is parainfluenza, E.R. is equine rhinovirus, HSV-1 and HSV-2 are Herpes simplex viruses types 1 and 2 and Vacc. is vaccinia virus.

TABLE 2

| Compound | ID$_{50}$ Vero 72 hrs. | McIntyre ED$_{90}$ | .T | MS ED$_{90}$ | .T | VSV ED$_{90}$ | .T | Col ED$_{90}$ | .T |
|---|---|---|---|---|---|---|---|---|---|
| Phyllanthoside | 1.19511 | 1.8667 | 0.6402 | 1.4958 | 0.7989 | NS | NC | NS | NC |
| Phyllanthocin | 5.90991 | 7.1380 | 0.8279 | 7.9939 | 0.7393 | 8.66792 | 0.6818 | 61.7506 | 0.0957 |
| Phyllanthostatin | 1.21212 | 2.3743 | 0.5105 | 1.16380 | 0.7399 | NS | NC | NS | NC |

| Compound | ID$_{50}$ Vero 91 hrs. | McIntyre ED$_{90}$ | .T | MS ED$_{90}$ | .T | VSV ED$_{90}$ | .T | Col ED$_{90}$ | .T |
|---|---|---|---|---|---|---|---|---|---|
| Phyllanthoside | 3.56835 | 1.8667 | 1.9115 | 1.4958 | 2.3854 | NS | NC | NS | NC |
| Phyllanthocin | 7.08898 | 7.1380 | 0.9931 | 7.9939 | 0.8867 | 8.66792 | 0.8178 | 61.7506 | 0.1148 |
| Phyllanthostatin | 3.63532 | 2.3743 | 1.5310 | 1.6380 | 2.2193 | NS | NC | NS | NC |

T - ID$_{50}$/ED90
NS - not significant
NC - not calculated

## TABLE 3

Protection of Female Mice from Lethal HSV-2 Inoculation
of the Vagina by Phyllanthostatins.

$8.0 \times 10^4$ PFU/0.1 ml

| Drug | mg/ml | N | %S | pa | MDD | pb |
|------|-------|---|-----|-----|-----|-----|
| Saline | - | 14 | 21 | | 7.0 | |
| Zn(OAc)$_2$ | 44 | 14 | 71 | 0.011 | 8.3 | 0.006 |
| Phyllanthoside | 1 | 14 | 64 | 0.027 | 9.0 | 0.003 |
| Phyllanthocin | 1 | 14 | 14 | 0.500 | 8.0 | 0.752 |
| Phyllanthostatin I | 1 | 14 | 29 | 0.500 | 7.4 | 0.770 |

$8.0 \times 10^3$ PFU/0.1 ml

| Drug | mg/ml | N | %S | pa | MDD | pb |
|------|-------|---|-----|-----|-----|-----|
| Saline | | 14 | 14 | | 9.0 | |
| Zn(OAc)$_2$ | | 14 | 79 | 0.001 | 3.0 | 0.024 |
| Phyllanthoside | | 14 | 93 | 0.001 | 14.0 | 0.001 |
| Phyllanthocin | | 14 | 29 | 0.329 | 8.5 | 0.935 |
| Phyllanthostatin I | | 14 | 71 | 0.003 | 11.8 | 0.002 |

---

a - Fisher exact test
b - Chi square analysis

TABLE 4

Frequency and Mean (10 G) Titers of Virus in Vaginal Secretions of Mice Described in Figures 2 and 3.

| Compound | Days After HSV-2 Inoc. | Nv/Nt[a] | Mean |
|---|---|---|---|
| Saline | 1 | 9/10 | 3.93 |
| | 2 | 10/10 | 4.44 |
| | 3 | 10/10 | 4.32 |
| | 4 | 10/10 | 4.30 |
| | 7 | 2/2 | 3.79 |
| $Zn(OAc)_2$ | 1 | 1/10 | (2.60) |
| | 2 | 1/10 | (3.54) |
| | 3 | 1/10 | (3.42) |
| | 4 | 1/9 | (4.54) |
| | 7 | 0/8 | -- |
| DMSO Saline | 1 | 9/10 | 3.50 |
| | 2 | 9/10 | 4.45 |
| | 3 | 9/10 | 4.25 |
| | 4 | 9/10 | 4.27 |
| | 7 | 2/3 | 4.01 |
| Phyllanthoside 1 mg/ml | 1 | 2/10 | 2.83 |
| | 2 | 3/10 | 2.98 |
| | 3 | 5/10 | 3.11 |
| | 4 | 4/10 | 3.72 |
| | 7 | 3/8 | 2.94 |
| Phyllanthoside 0.5 mg/ml | 1 | 5/10 | 2.46 |
| | 2 | 7/10 | 3.45 |
| | 3 | 8/10 | 3.41 |
| | 4 | 8/10 | 3.73 |
| | 7 | 2/4 | 3.98 |
| Phyllanostatin I 1 mg/ml | 1 | 6/10 | 1.67 |
| | 2 | 8/10 | 3.83 |
| | 3 | 8/10 | 3.93 |
| | 4 | 8/10 | 3.63 |
| | 7 | 3/5 | 3.47 |

| Compound | Days After HSV-2 Inoc. | Nv/Nt[a] | Mean |
|---|---|---|---|
| Phyllanostatin I 0.5 mg/ml | 1 | 9/10 | 2.69 |
| | 2 | 10/10 | 3.76 |
| | 3 | 10/10 | 3.71 |
| | 4 | 10/10 | 4.13 |
| | 7 | 1/1 | (3.98) |

a - NV/NT is the number of swabs with virus/the total number of swabs in the group

b - mean is the means of the virus titers in the swabs of those mice which shed virus.

-22-

CLAIMS

1. The use of a compound selected from phyllanthostatin I, phyllanthoside and phyllanthocin, for the manufacture of a medicament for treating or preventing a virus in a mammal suffering from or susceptible to the virus.

2. The use according to calim 1, wherein the compound is phyllanthostatin I.

3. The use according to claim 1, wherein the compound is phyllanthoside.

4. The use according to claim 1, wherein the compound is phyllanthocin.